(19) Europäisches Patentamt
European Patent Office
Office européen des brevets

(11) Veröffentlichungsnummer: **0 409 118 B1**

# (12) EUROPÄISCHE PATENTSCHRIFT

(45) Veröffentlichungstag der Patentschrift: **12.10.94**

(51) Int. Cl.$^5$: **C07D 231/08**, A01N 43/56, A01N 25/32

(21) Anmeldenummer: **90113526.9**

(22) Anmeldetag: **14.07.90**

## (54) Pyrazoline und ihre Verwendung als Safener.

(30) Priorität: **18.07.89 DE 3923649**

(43) Veröffentlichungstag der Anmeldung:
**23.01.91 Patentblatt 91/04**

(45) Bekanntmachung des Hinweises auf die Patenterteilung:
**12.10.94 Patentblatt 94/41**

(84) Benannte Vertragsstaaten:
**BE CH DE ES FR GB IT LI NL**

(56) Entgegenhaltungen:
**EP-A- 0 333 131**
**DE-A- 2 603 400**
**GB-A- 1 025 430**

**CHEMICAL ABSTRACTS, Band 85, Nr. 19, 8. November 1976, Seite 519, Zusammenfassung Nr. 143027e, Columbus, Ohio, US; Y.I. KHERUZE et al.: "1,3-Dipolar cycloaddition to unsaturated compounds. Reaction of 2-methoxy-1,3-abutadiene with nitrylimines"**

**CHEMICAL ABSTRACTS, Band 89, Nr. 21, 20. November 1978, Seite 600, Zusammenfassung Nr. 179912m, Columbus, Ohio, US; Y.I. KHERUZE et al.:"Reactions of 2-alkoxy-1,3-butadienes with nitrilimines"**

(73) Patentinhaber: **Hoechst Schering AgrEvo GmbH**
**Gerichtstrasse 27**
**D-13342 Berlin (DE)**

(72) Erfinder: **Sohn, Erich, Dr.**
**Lerchenbergstrasse 46/1**
**D-7300 Esslingen (DE)**
Erfinder: **Rösch, Wolfgang, Dr.**
**Geisenheimer Strasse 92**
**D-6000 Frankfurt am Main (DE)**
Erfinder: **Bauer, Klaus, Dr.**
**Doorner Strasse 53d**
**D-6450 Hanau (DE)**
Erfinder: **Bieringer, Hermann, Dr.**
**Eichenweg 26**
**D-6239 Eppstein/Taunus (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 409 118 B1

**Beschreibung**

Bei der Anwendung von Pflanzenbehandlungsmitteln, insbesondere von Herbiziden, können unerwünschte, nicht tolerierbare Schäden an Kulturpflanzen auftreten. Besonders bei der Applikation von Herbiziden nach dem Auflaufen der Kulturpflanzen besteht daher oft das Bedürfnis, das Risiko einer möglichen Phytotoxizität zu vermeiden.

Solche Verbindungen, die die Eigenschaften besitzen, Kulturpflanzen gegen phytotoxische Schäden durch Herbizide zu schützen, ohne die eigentliche herbizide Wirkung dieser Mittel zu beeinträchtigen, werden "Antidot" oder "Safener" genannt.

Verschiedene Verbindungen wurden für diese Anwendung bereits beschrieben; vgl. z.B. EP-A 152 006 (= US-A-4,602,932) und EP-A 0174562 (= US-A-4,639,266).

In der Deutschen Patentanmeldung P-3 808 896.7 (EP-A 0 333 131) wurden 1-Phenyl- und 1-(Pyrid-2-yl)pyrazolderivate als Safener vorgeschlagen.

Nur wenige 1-Aryl-4,5-pyrazolin-3-carbonsäurederivate sind bekannt. So beschreiben Kheraze et al. (Zh. Org. Khim. 12 (1976) 6, 1332-1337 und Zh. Org. Khim. 14 (1978) 1396-1401) Pyrazoline der Formeln

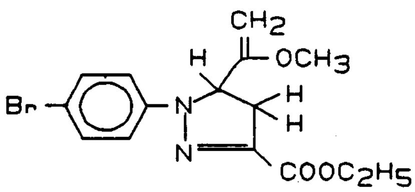

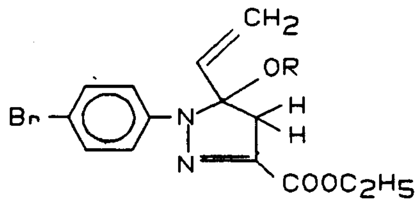

R = $CH_3$ oder i-Propyl

Eine biologische Wirkung dieser Alkoxypyrazoline wurde bisher nicht beschrieben.

Gegenstand der vorliegenden Erfindung sind 4,5-Pyrazolin-3-carbonsäurederivateder Formel (I)

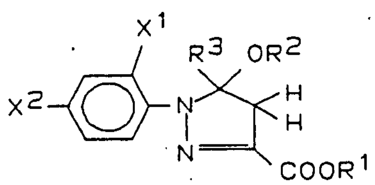

(I)

worin

$X^1$ und $X^2$ unabhängig voneinander Halogen oder Halogenalkyl,
$R^1$ Wasserstoff, Alkyl, Cycloalkyl oder Alkoxyalkyl,
$R^2$ Alkyl, Cycloalkyl oder Halogenalkyl und
$R^3$ Wasserstoff, Alkyl, Cycloalkyl, Alkenyl oder Alkinyl bedeuten.

Die Formel (I) umfaßt dabei alle möglichen geometrischen Isomeren und Stereoisomeren. Weiterhin bedeuten in Formel (I) Halogen Fluor, Chlor, Brom oder Jod, Alkyl geradkettiges oder verzweigtes Alkyl, Alkenyl geradkettiges oder verzweigtes Alkenyl, wobei die

Doppelbindung an beliebiger Stelle im Alkenylrest vorliegen kann und Alkinyl geradkettiges oder verzweigtes Alkinyl, wobei auch hier die Dreifachbindung beliebig im Alkinylrest lokalisiert sein kann, Halogenalkyl ein- oder mehrfach durch Halogen substituiertes Alkyl.

Von besonderem Interesse sind erfindungsgemäße Verbindungen der Formel (I), worin

$X^1$ und $X^2$ unabhängig voneinander Halogen oder $C_1$-$C_4$-Halogenalkyl,
$R^1$ $C_1$-$C_6$-Alkyl, $C_3$-$C_6$-Cycloalkyl oder ($C_1$-$C_6$-Alkyloxy)-$C_1$-$C_6$-alkyl,
$R^2$ $C_1$-$C_6$-Alkyl, $C_3$-$C_6$-Cycloalkyl oder $C_1$-$C_6$-Halogenalkyl und
$R^3$ Wasserstoff, $C_1$-$C_6$-Alkyl, $C_3$-$C_6$-Cycloalkyl, $C_2$-$C_6$-Alkenyl oder $C_2$-$C_6$-Alkinyl
bedeuten.

Haloalkyl bedeutet bevorzugt Trifluormethyl, 2-Chlorethyl, 1,1,2,2-Tetrafluorenyl oder Hexafluorenyl, Halogen bevorzugt Fluor, Chlor oder Brom.

Alkyl steht bevorzugt für einen der Reste Methyl, Ethyl, n-Propyl, i-Propyl, 1-Propen-2-yl, die Butyl-, Pentyl- und Hexylisomeren.

Cycloalkyl steht bevorzugt für Cyclopentyl und Cyclohexyl. Alkenyl bedeutet bevorzugt einen der Reste Vinyl, 1-Propen-1-yl, 1-Propen-2-yl, die Butenyl-, Pentenyl- und Hexenylisomeren.
Alkinyl steht bevorzugt für Acetylenyl, 1-Propinyl oder 2-Propinyl.

Besonders bevorzugt sind erfindungsgemäße Verbindungen der Formel (I), worin

| | |
|---|---|
| $X^1$ und $X^2$ | unabhängig voneinander Fluor, Chlor, Brom oder Trifluormethyl, |
| $R^1$ | $C_1$-$C_4$-Alkyl, ($C_1$-$C_4$-Alkyloxy)-$C_1$-$C_4$-alkyl, |
| $R^2$ | $C_1$-$C_4$-Alkyl, das unsubstituiert oder ein- oder mehrfach durch Halogen substituiert ist, und |
| $R^3$ | Wasserstoff, $C_1$-$C_4$-Alkyl, $C_2$-$C_4$-Alkenyl oder $C_2$-$C_4$-Alkinyl |

bedeuten.

Gegenstand der Erfindung ist ferner ein Verfahren zur Herstellung der Verbindungen der Formel (I), dadurch gekennzeichnet, daß man eine Verbindung der Formel (II)

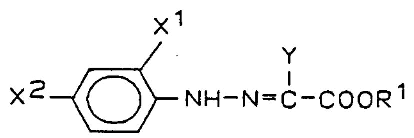
(II)

worin Y für Chlor oder Brom steht und $X^1$, $X^2$ und $R^1$ die oben angegebenen Bedeutungen haben, mit Enolethern der Formel (III)

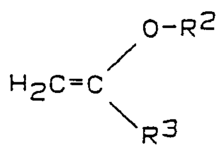
(III)

worin $R^2$ und $R^3$ die oben angegebenen Bedeutungen haben, umsetzt.

Die Komponenten können äquimolar oder im Überschuß der Verbindungen der Formel (III) eingesetzt werden, üblicherweise im molaren Verhältnis (II) : (III) von 1 : 1,05 bis 1 : 20, bevorzugt im molaren Verhältnis 1 : 1,1 bis 1 : 5.

Die Verbindungen der Formel (II) sind zum Teil bekannt oder können nach üblichen Verfahren synthetisiert werden. Sie lassen sich beispielsweise aus den entsprechenden Anilinen durch Diazotieren und Kuppeln mit den entsprechenden 2-Chlor-acetessigestern erhalten. Die Verbindungen der Formel (III) sind ebenfalls nach üblichen Verfahren zugänglich, zum Beispiel durch Alkoholabspaltung aus den entsprechenden Ketalen.

Die Umsetzung wird in der Regel zwischen 0 und 150 °C, vorteilhaft zwischen 20 und 100 °C, gegebenenfalls in Gegenwart einer organischen Base, wie sterisch gehinderte Amine, z.B. Triethylamin oder Pyridin, oder einer anorganischen Base, wie z.B. Kaliumcarbonat, Kaliumhydroxyd oder Natriumcarbonat, mit oder ohne Gegenwart eines organischen Lösungsmittels, wie gegebenenfalls eines halogenierten aliphatischen oder aromatischen Kohlenwasserstoffs oder eines Ethers, beispielsweise des Lösungsmittels Toluol, Xylol, Dichlorethan, Dimethoxyethan, Di- oder Triglyme, Cyclohexan, Petrolether oder Chlorbenzol, durchgeführt. Basen und Lösungsmittel sind nur beispielhaft aufgezählt, ohne daß das Verfahren auf diese Beispiele beschränkt ist.

Die Verbindungen der Formel (I) lassen sich durch Alkoholabspaltung in entsprechende Pyrazolester, wie sie in der Deutschen Patentanmeldung P 3 808 896.7 (EP-A-0 333 131) als Safener beschrieben sind, überführen.

Gegenstand der Erfindung ist deshalb ferner ein Verfahren zur Umwandlung der Verbindungen der Formel (I) in die entsprechenden Pyrazole, dadurch gekennzeichnet, daß die Verbindungen der Formel (I) ohne oder in Gegenwart eines organischen Lösungsmittels, wie z.B. eines gegebenenfalls halogenierten aliphatischen oder aromatischen Kohlenwasserstoffs oder eines Ethers, beispielsweise des Lösungsmittels Toluol, Xylol, Chlorbenzol oder Di- oder Triglyme, gegebenenfalls in Gegenwart einer anorganischen oder organischen Säure, wie Mineralsäuren oder organische Sulfonsäuren, beispielsweise der Säuren p-Toluolsulfonsäure, Phosphorsäure, Schwefelsäure oder Trifluormethansulfonsäure, bei Temperaturen zwischen 0 und 200 °C, vorteilhaft zwischen 50 und 120 °C, unter Abspaltung der Verbindung der Formel $HOR^2$, worin $R^2$ die in Formel (I) definierte Bedeutung hat, behandelt werden.

Die Verbindungen der Formel (I) haben die Eigenschaft, phytotoxische Nebenwirkungen von Pflanzenschutzmitteln, insbesondere von Herbiziden, beim Einsatz in Nutzpflanzenkulturen zu vermindern oder ganz

zu verhindern. Die Verbindungen der Formel (I) sind in der Lage, schädliche Nebenwirkungen der Herbizide weitgehend oder völlig aufzuheben, ohne die Wirksamkeit dieser Herbizide gegen Schadpflanzen zu schmälern. Das Einsatzgebiet herkömmlicher Herbizide kann durch Zugabe der Safenerverbindung der Formel (I) ganz erheblich vergrößert werden.

Gegenstand der vorliegenden Erfindung ist daher auch ein Verfahren zum Schutz von Kulturpflanzen gegen phytotoxische Nebenwirkungen von Pflanzenschutzmitteln, insbesondere Herbiziden, das dadurch gekennzeichnet ist, daß man die Pflanzen, Pflanzensamen oder Anbauflächen mit einer Verbindung der Formel (I) vor, nach oder gleichzeitig mit dem Pflanzenschutzmittel behandelt.

Herbizide, deren phytotoxische Nebenwirkungen mittels der Verbindungen der Formel (I) herabgesetzt werden können sind z.B. Carbamate, Thiocarbamate, Halogenacetanilide, substituierte Phenoxy-, Naphthoxy- und Phenoxyphenoxycarbonsäurederivate sowie Heteroaryloxyphenoxycarbonsäurederivate wie Chinolyloxy-, Chinoxalyloxy-, Pyridyloxy-, Benzoxazolyloxy-, Benzthiazolyloxy-phenoxy-carbonsäureester und ferner Dimedonoximabkömmlinge. Bevorzugt hiervon sind Phenoxyphenoxy- und Heteroaryloxyphenoxycarbonsäureester und strukturelle Analoga wie Benzylphenoxycarbonsäureester. Als Ester kommen hierbei insbesondere niedere Alkyl-, Alkenyl- und Alkinylester in Frage.

Beispielsweise seien, ohne daß dadurch eine Beschränkung erfolgen soll, folgende Herbizide genannt:

A) Herbizide vom Typ der Phenoxyphenoxy- und Heteroaryloxyphenoxycarbonsäure-($C_1$-$C_4$)-alkyl-, ($C_2$-$C_4$)-alkenyl)- oder ($C_3$-$C_4$)-alkinylester wie 2-(4-(2,4-Dichlorphenoxy)-phenoxy)-propionsäuremethylester, 2-(4-(4-Brom-2-chlorphenoxy)-phenoxy)-propionsäuremethylester, 2-(4-(4-Trifluormethylphenoxy)-phenoxy)-propionsäuremethylester, 2-(4-(2-Chlor-4-trifluormethylphenoxy)-phenoxy)-propionsäuremethylester, 2-(4-(2,4-Dichlorbenzyl)phenoxy)-propionsäuremethylester, 2-Isopropylideneaminooxyethyl-(R)-2-[4-(6-chloroquinoxalin-2-yloxy)-phenoxy]propionat (Propaquizafop), 4-(4-(4-Trifluormethylphenoxy)-phenoxy)-pent-2-ensäureethylester, 2-(4-(3,5-Dichlorpyridyl-2-oxy)phenoxy)-propionsäureeethylester, 2-(4-(3,5-Dichlorpyridyl-2-oxy)-phenoxy)-propionsäurepropargylester, 2-(4-(6-Chlorbenzoxazol-2-yloxy)-phenoxy)propionsäureethylester, 2-(4-(6-Chlorbenzthiazol-2-yl-oxy)-phenoxy)-propionsäureethylester, 2-(4-(3-Chlor-5-trifluormethyl-2-pyridyloxy)-phenoxy)propionsäuremethylester, 2-(4-(5-Trifluormethyl-2-pyridyloxy)-phenoxy)-propionsäurebutylester, 2-(4-(6-Chlor-2-chinoxalyloxy)-phenoxy)-propionsäureethylester, 2-(4-(6-Fluor-2-chinoxalyloxy)-phenoxy)propionsäureethylester, 2-(4-(5-Chlor-3-fluorpyridyl-2-oxy)-phenoxy)-propionsäurepropargylester, 2-(4-(6-Chlor-2-chinolyloxy)-phenoxy)-propionsäureethylester, 2-(4-(3,5-Dichlorpyridyl-2-oxy)-phenoxy) propionsäuretrimethylsilylmethylester, 2-(4-(3-Chlor-5-trifluormethoxy-2pyridyloxy)-phenoxy)propionsäureethylester,

B) Chloracetanilid-Herbizide wie N-Methoxymethyl-2,6-diethyl-chloracetanilid, 2-chlor-N-(2-ethyl-6-methylphenyl)-N-(2-methoxy-1-methylethyl)-acetamid, N-(3-Methyl-1,2,4-oxdiazol-5-yl-methyl)chloressigsäure-2,6-dimethylanilid,

C) Thiocarbamate wie S-Ethyl-N,N-dipropylthiocarbamat oder S-Ethyl-N,N-diisobutylthiocarbamat,

D) Dimedon-Derivate wie 2-(N-Ethoxybutyrimidoyl)-5-(2-ethylthiopropyl)-3-hydroxy-2-cyclohexen-1-on, 2-(N-Ethoxybutyrimidoyl)-5-(2-phenylthiopropyl)-3-hydroxy-2-cyclohexen-1-on oder 2-(1-Allyloxyiminobutyl)-4-methoxycarbonyl-5,5-dimethyl-3-oxocyclohexenol, 2-(N-Ethoxypropionamidoyl)-5-mesityl-3-hydroxy-2-cyclohexen-1-on (oder auch als 5-(2,4,6-Trimethylphenyl)-3-hydroxy-2-[1-(Ethoxyimino)propyl]-cyclohex-2-en-1-on bezeichnet), 2-(N-Ethoxybutyrimidoyl)-3-hydroxy-5-(thian-3-yl)-2-cyclohexen-1-on, 2-[1-Ethoxyimino)-butyl]-3-hydroxy-5-(2H-tetrahydrothiopyran-3-yl)-2-cyclohexen-1-on (BASF 517); (±)-2-[-(E)-3-Chlorallyloxyiminopropyl]-5-(2-ethylthiopropyl)-3-hydroxycyclohex-2-enon (Clethodim).

Von den Herbiziden, welche erfindungsgemäß mit den Verbindungen der Formel (I) kombiniert werden können, sind bevorzugt die unter A) aufgeführten Verbindungen zu nennen, insbesondere 2-(4-(6-Chlorbenzoxazol-2-yl-oxy)-phenoxy)propionsäureethylester, 2-(4-(6-Chlorbenzthiazol-2-yl-oxy)phenoxy)-propionsäureethylester und 2-(4-(5-Chlor-3-fluorpyridyl-2-oxy)-phenoxy)-propionsäurepropargylester. Von den unter D) genannten Substanzen ist insbesondere 2-(N-Ethoxypropionamidoyl)-5-mesityl-3-hydroxy-2-cyclohexen-1-on von Bedeutung.

Das Gewichtsverhältnis Safener (Verbindung I) : Herbizid kann innerhalb weiter Grenzen variieren und ist vorzugsweise im Bereich von 1 : 10 bis 10 : 1, insbesondere 2 : 1 bis 1 : 10.

Die jeweils optimalen Mengen an Herbizid und Safener sind abhängig vom Typ des verwendeten Herbizids oder vom verwendeten Safener sowie von der Art des zu behandelnden Pflanzenbestandes und lassen sich von Fall zu Fall durch entsprechende Versuche ermitteln.

Haupteinsatzgebiete für die Anwendung der Safener sind vor allem Getreidekulturen (Weizen, Roggen, Gerste, Hafer), Reis, Mais, Sorghum, aber auch Baumwolle, Zuckerrüben, Zuckerrohr und Sojabohne.

Die Safener können je nach ihren Eigenschaften zur Vorbehandlung des Saatgutes der Kulturpflanze (Beizung der Samen) verwendet werden oder vor der Saat in die Saatfurchen eingebracht werden oder zusammen mit dem Herbizid vor oder nach dem Auflaufen der Pflanzen angewendet werden. Vorauflaufbehandlung schließt sowohl die Behandlung der Anbaufläche vor der Aussaat als auch die Behandlung der angesäten, aber noch nicht bewachsenen Anbauflächen ein.

Bevorzugt ist jedoch die gleichzeitige Anwendung des Antidots mit dem Herbizid in Form von Tankmischungen oder Fertigformulierugen.

Die Verbindungen der Formel (I) oder deren Kombination mit einem oder mehreren der genannten Herbizide bzw. Herbizidgruppen können auf verschiedene Art formuliert werden, je nachdem wie es durch die biologischen und/oder chemisch-physikalischen Parameter vorgegeben ist. Als Formulierungsmöglichkeiten kommen daher infrage: Spritzpulver (WP), emulgierbare Konzentrate (EC), wäßrige Lösungen (SL), konzentrierte Emulsionen (EW) wie Öl-in-Wasser- und Wasser-in-Öl-Emulsionen, versprühbare Lösungen oder Emulsionen, Dispersionen auf Öl- oder Wasserbasis (SC), Stäubemittel (DP), Beizmittel, Granulate in Form von Mikro-, Sprüh-, Aufzugs- und Adsorptionsgranulaten, Boden- und Streu-Granulate, wasserdispergierbare Granulate (WG), ULV-Formulierungen, Mikrokapseln oder Wachse.

Diese einzelnen Formulierungstypen sind im Prinzip bekannt und werden beispielsweise beschrieben in: Winnacker-Küchler, "Chemische Technologie", Band 7, C. Hauser Verlag, München, 4. Auflage 1986; van Valkenburg, "Pesticides Formulations", Marcel Dekker N.Y., 2nd Ed. 1972-73; K. Martens, "Spray Drying Handbook", 3rd Ed. 1979, G. Goodwin Ltd, London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldewell N.J.; H. v. Olphen, "Introduction to Clay Colloid Chemistry", 2nd Ed., J. Wiley & Sons, N.Y. 1950; McCutcheon's, "Detergents and Emulsifiers Annual", MC Publ. Corp., Ridgewood N.J.; Sisley and Wood, "Encyclopedia of Suface Active Agents", Chem. Publ. Co. Inc., N.Y. 1964; Schönfeldt, "Grenzflächenaktive Äthylenoxidaddukte", Wiss. Verlagsgesellschaft, Stuttgart 1976; Winnacker-Küchler, "Chemische Technologie", Band 7, C. Hauser Verlag München, 4. Aufl. 1986.

Gegenstand der Erfindung sind deshalb auch die Mittel, welche die erfindungsgemäßen Verbindungen der Formel (I) enthalten. Vor allem sind diese einerseits pflanzenschützende Mittel, die einen oder mehrere Verbindungen der Formel (I) und dem jeweiligen Formulierungstyp entsprechende übliche inerte Hilfsmittel enthalten, und andererseits herbizide Mittel, die eine Kombination von Verbindungen der Formel (I) und ein oder mehrere Herbizide und dem jeweiligen Formulierungstyp entsprechende übliche Hilfsmittel enthalten.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole und Fettamine, Alkansulfonate oder Alkylarylsulfonate, und Dispergiermittel, z.B. ligninsulfonsaures Natrium, 2,2′-dinaphthylmethan-6,6′-disulfonsaures Natrium, dibutylnaphthalin-sulfonsaures Natrium oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte (z.B. Blockpolymere), Alkylpolyether, Sorbitanfettsäureester, Polyoxyethylensorbitanfettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Pyrophillit oder Diatomeenerde.

Granulate können entweder durch Verdüsen des Wirkstoffes auf adsorptionsfähiges, granuliertes Intertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In der Regel enthalten die erfindungsgemäßen Formulierungen 0,1 bis 99 Gew.-%, vorzugsweise 1 bis 95 Gew.-%, insbesondere 2 bis 90 Gew.-% Wirkstoff, d.h. Wirkstoff der Formel (I) oder eine Kombination des Wirkstoffs der Formel (I) mit einem Herbizid.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentrate kann die Wirkstoffkonzentration etwa 1 bis 80 Gew.-%, vorzugsweise 5 bis 80 Gew.-% betragen. Staubförmige Formulierungen

enthalten meistens 1 bis 30 Gew.-%, vorzugsweise 5 bis 20 Gew.-% an Wirkstoff, versprühbare Lösungen etwa 0,2 bis 25 Gew.-%, vorzugsweise 2 bis 20 Gew.% Wirkstoff. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei wasserdispergierbaren Granulaten liegt der Wirkstoffgehalt in der Regel zwischen 10 und 90 Gew.-%

Daneben enthalten die genannten Wirkstofformulierungen gegebenenfalls die jeweils üblichen Haft-, Netz-, Dispergier-, Emulgier-, Penetrations-, Lösungsmittel, Füll-oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids u. a. variiert die erforderliche Aufwandmenge der Verbindungen der Formel (I). Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Folgende Beispiele dienen zur Erläuterung der Erfindung:

## A. Formulierungsbeispiele

a) Ein Stäubemittel wird erhalten, indem man 10 Gew.-Teile einer Verbindung der Formel (I) oder gegebenenfalls ein Wirkstoffgemisch mit einem Herbizid und 90 Gew.-Teile Talkum als Inertstoff mischt und in einer Schlagmühle zerkleinert.

b) Ein in Wasser leicht dispergierbares, benetzbares Pulver wird erhalten, indem man 25 Gew.-Teile einer Verbindung der Formel (I) oder eines Gemisches aus (I) mit einem Herbizid, 64 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gew.-Teile einer Verbindung der Formel (I) oder eines Gemisches aus (I) mit einem Herbizid, mit 6 Gew.-Teilen Alkylphenolpolyglykolether (®Triton X 207), 3 Gew.-Teilen Isotridecanolpolyglykolether (8 EO = 8 Ethylenoxyeinheiten) und 71 Gew.-Teilen paraffinischem Mineralöl (Siedebereich z.B. ca. 255 bis über 277 °C) mischt und in einer Reibkugelmühle auf eine Feinheit von unter 5 Mikron vermahlt.

d) Ein emulgierbares Konzentrat wird erhalten aus 15 Gew.-Teilen einer Verbindung der Formel (I) oder eines Gemisches aus (I) und einem Herbizid, 75 Gew.-Teilen Cyclohexanon als Lösungsmittel und 10 Gew.-Teilen oxethyliertes Nonylphenol als Emulgator.

e) Ein in Wasser leicht emulgierbares Konzentrat aus einem Phenoxycarbonsäureester und einem Antidot (10 : 1) wird erhalten aus
12,00 Gew.-% 2-[4-(6-Chlorbenzoxazol-2-yl-oxy)-phenoxy]propionsäureethylester,
1,20 Gew.-% Verbindung der Formel (I),
69,00 Gew.-% Xylol,
7,80 Gew.-% dodecylbenzolsulfonsaurem Calcium,
6,00 Gew.-% ethoxyliertem Nonylphenol (10 EO) und
4,00 Gew.-% ethoxyliertem Rizinusöl (40 EO).

Die Zubereitung erfolgt wie unter Beispiel a) angegeben.

f) Ein in Wasser leicht emulgierbares Konzentrat aus einem Phenoxycarbonsäureester und einem Antidot (1 : 10) wird erhalten aus
4,0 Gew.-% 2-[4-(6-Chlorbenzoxazol-2-yl-oxy)-phenoxy]propionsäureethylester,
40,0 Gew.-% Verbindung der Formel (I),
30,0 Gew.-% Xylol,
20,0 Gew.-% Cyclohexanon,
4,0 Gew.-% dodecylsulfonsaurem Calcium und
2,0 Gew.-% ethoxyliertem Rizinusöl (40 EO).

## B. Herstellungsbeispiele

**Beispiel 1:**

1-(2,4-Dichlorphenyl)-5-isopropyl-5-methoxy-pyrazolin-3-carbonsäureethylester

10 g 2-Methoxy-3-methyl-but-1-en und 10 g Triethylamin werden auf 80 °C erwärmt. Innerhalb von einer halben Stunde läßt man zu dieser Mischung 15,5 g des 2,4-Dichlorphenylhydrazons von 2-Chlorglyoxalsäureethylester (Formel (II) mit $X^1 = X^2 = Y = Cl$, $R^1 = C_2H_5$) (IIa) in 50 ml Toluol zutropfen. Man rührt 4 h bei 80 °C nach, saugt nach Abkühlen vom Niederschlag ab und engt im Vakuum schonend ein. Nach Säulenchromatographie (Laufmittel Petrolether/Essigester 1 : 0 --> 1 : 5) über Kieselgel erhält man 8,3 g Pyrazolin als Öl.

**Beispiel 2:**

1-(2,4-Dichlorphenyl)-5-ethoxy-5-isopropyl-pyrazolin-3-carbonsäureethylester

6,2 g 2-Ethoxy-3-methyl-but-1-en und 6 g Triethylamin werden in 20 ml Cyclohexan bei 70 °C vorgelegt; während 1/2 h läßt man 15,0 g der Verbindung (IIa) nach Beispiel 1 in 150 ml Cyclohexan zutropfen. Nach 10 h bei dieser Temperatur wird vom Niederschlag abgesaugt und im Vakuum eingeengt. Aus der Mutterlauge fällt ein Niederschlag des Produkts (9,2 g) vom Schmelzpunkt 105 - 110 °C aus.

**Beispiel 3:**

1-(2,4-Dichlorphenyl)-5-methoxy-5-t-butyl-pyrazolin-3-carbonsäureethylester

22,8 g 3,3-Dimethyl-2-methoxy-but-1-en, 15 g Kaliumcarbonat und 20 ml Dimethoxyethan werden auf 80 °C erwärmt; bei derselben Temperatur läßt man 15 g der Verbindung (IIa) nach Beispiel 1 in 100 ml Toluol während 1/2 h zutropfen. Nach 15 h Nachrühren wird vom Niederschlag abgesaugt, im Vakuum schonend eingeengt und wie im Beispiel 1 chromatographiert. Man erhält 10,7 g Produkt vom Schmelzpunkt 130 - 133 °C.

Analog können die Beispiele der Tabelle I hergestellt werden.

**Tabelle I: Alkoxypyrazoline der Formel (I)**

| Beisp.-Nr. | $X^1$, $X^2$ | $R^1$ | $R^2$ | $R^3$ | Fp [°C] |
|---|---|---|---|---|---|
| 4 | 2,4-$Cl_2$ | $C_2H_5$ | n-$C_4H_9$ | H | Oel |
| 5 | " | $CH_3$ | $CH_3$ | i-$C_3H_7$ | |
| 6 | " | $(CH_2)_2OCH_3$ | $CH_3$ | i-$C_3H_7$ | 76-78 |
| 7 | " | $C_2H_5$ | $CH_3$ | $C_2H_3$ | |
| 8 | " | $C_2H_5$ | $CH_3$ | $C_2H$ | Harz |
| 9 | 4-Cl-2-$CF_3$ | $C_2H_5$ | $CH_3$ | i-$C_3H_7$ | Harz |
| 10 | 2,4-$Br_2$ | $C_2H_5$ | $CH_3$ | i-$C_3H_7$ | Oel |
| 11 | 2-Cl-4-$CF_3$ | $C_2H_5$ | $CH_3$ | i-$C_3H_7$ | |
| 12 | 2,4-$Cl_2$ | $C_2H_5$ | $CH_3$ | $CH_3$ | |
| 13 | 2,4-$Cl_2$ | n-$C_3H_7$ | $C_2H_5$ | H | |
| 14 | " | n-$C_4H_9$ | $CH_3$ | H | |
| 15 | " | n-$C_5H_{11}$ | $CH_3$ | $CH_3$ | |
| 16 | " | i-$C_6H_{13}$ | i-$C_3H_7$ | H | |
| 17 | " | Cyclohexyl | $CH_3$ | i-$C_3H_7$ | |
| 18 | " | Cyclopentyl | $CH_3$ | Vinyl | |
| 19 | " | $CH_3$ | n-$C_5H_{11}$ | -C≡CH | |
| 20 | " | $CH_3$ | i-$C_6H_{13}$ | -C≡CH | |
| 21 | " | $(CH_2)_3OCH_3$ | $CH_3$ | $C_2H_5$ | |
| 22 | " | $(CH_2)_2OC_2H_5$ | $C_2H_5$ | $C_2H_5$ | |
| 23 | 2,4-$(CF_3)_2$ | $CH_3$ | $CH_3$ | $CH_3$ | |
| 24 | " | $C_2H_5$ | $CH_3$ | $CH_3$ | |
| 25 | " | $C_2H_5$ | $CH_3$ | i-$C_3H_7$ | |
| 26 | 2,4-$F_2$ | $CH_3$ | $CH_3$ | H | |
| 27 | 2-F-4-Cl | $CH_3$ | $CH_3$ | H | |
| 28 | 2-F-4-$CF_3$ | $C_2H_5$ | $C_2H_5$ | H | |
| 29 | 2,4-$Br_2$ | $(CH_2)_2$-$OCH_3$ | $CH_3$ | Vinyl | |
| 30 | 4-Cl-2-$CF_3$ | $CH_3$ | $C_2H_5$ | H | |
| 31 | 2,4-$Cl_2$ | $CH_3$ | $CH_3$ | Cyclohexyl | |
| 32 | " | $C_2H_5$ | $CH_3$ | Cyclopentyl | |
| 33 | " | $CH_3$ | $C_2H_5$ | Vinyl | |
| 34 | " | $C_2H_3$ | 2-$ClC_2H_4$ | H | |
| 35 | " | $C_2H_5$ | 2-$ClC_2H_4$ | H | |
| 36 | " | $C_2H_5$ | 3-H-$C_3F_6$ | H | |

## C. Beispiele für Umwandlung in Pyrazole

### Beispiel 1:

1-(2,4-Dichlorphenyl)-5-isopropyl-pyrazol-3-carbonsäureethylester

10 g Pyrazolin aus Beispiel B.1 werden 2 h bei 100 °C gehalten und aus wenig Petrolether umkristallisiert. Es fällt 6,3 g Produkt mit einem Schmelzpunkt von 93 - 95 °C aus (siehe Hinweis am Ende von Beispiel 2).

### Beispiel 2:

10 g Rohprodukt aus Beispiel B.1 (vor Säulenchromatographie) werden in 150 ml wasserfreiem Toluol aufgenommen und mit 0,5 g p-Toluolsulfonsäure 4 h am Rückfluß gekocht. Man engt im Vakuum ein und kristallisiert aus wenig Petrolether um. Ausbeute 6,1 g, Fp. 93 - 95 °C (Hinweis: Der in P 3 808 896.7, Beispiel 302 angegebene niedrigere Schmelzpunkt von 70 - 77 °C ist auf Verunreinigung mit dem isomeren Pyrazol-5-ester zurückzuführen.)

## D. Biologische Beispiele

### Beispiel 1:

Weizen und Gerste wurden im Gewächshaus in Plastiktöpfen bis zum 3- bis 4-Blattstadium herangezogen und dann nacheinander mit den Safener-Verbindungen und den getesteten Herbiziden im Nachauflaufverfahren behandelt. Die Herbizide und die Verbindungen der Formel (I) wurden dabei in Form wäßriger Suspensionen bzw. Emulsionen mit einer Wasseraufwandmenge von umgerechnet 800 l/ha ausgebracht. 3 bis 4 Wochen nach der Behandlung wurden die Pflanzen visuell auf jede Art von Schädigung durch die ausgebrachten Herbizide bonitiert, wobei insbesondere das Ausmaß der anhaltenden Wachstumshemmung berücksichtigt wurde. Der Grad der Schädigung bzw. die Safener-Wirkung von Verbindungen der Formel (I) alleine bzw. in Kombination mit Herbiziden wurde in % Schädigung bestimmt.

Die Ergebnisse zeigen (vgl. Tabelle II), daß die erfindungsgemäßen Verbindungen starke Herbizidschäden an Kulturpflanzen effektiv reduzieren können.

Selbst bei starken Überdosierungen eines Herbizids wie Fenoxaprop-ethyl werden bei Kulturpflanzen auftretende schwere Schädigungen deutlich reduziert, geringere Schäden völlig aufgehoben. Mischungen aus Herbiziden und erfindungsgemäßen Verbindungen eignen sich deshalb in vorteilhafter Weise zur selektiven Unkrautbekämpfung in Getreidekulturen.

Tabelle II

| **Safenerwirkung** | | | |
|---|---|---|---|
| Wirkstoff/W.-gemisch | Dosis [kg a.i./ha] | Herbizide Wirkung in % | |
| | | TRAE | HOVU |
| H | 2,0 | 75 | - |
| | 0,2 | - | 80 |
| H + Bsp. 1 | 2,0 + 1,0 | 10 | - |
| | 2,0 + 0,25 | 10 | - |
| | 0,2 + 1,0 | - | 5 |
| | 0,2 + 0,25 | - | 10 |
| H + Bsp. 10 | 0,2 + 1,25 | - | 17 |

Abkürzungen: H = Herbizid Fenoxaprop-ethyl
Bsp. Nr. = siehe Beispiel aus Tabelle I
TRAE = Triticum aestivum (Weichweizen)
HOVU = Hordeum vulgare (Gerste)

**Patentansprüche**
**Patentansprüche für folgende Vertragsstaaten : BE, CH, DE, FR, GB, IT, LI, NL**

1. Verbindungen der Formel (I)

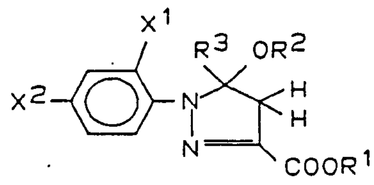

(I)

worin
$X^1$ und $X^2$ unabhängig voneinander Halogen oder Halogenalkyl,
$R^1$ Wasserstoff, Alkyl, Cycloalkyl oder Alkoxyalkyl,
$R^2$ Alkyl, Cycloalkyl oder Halogenalkyl und
$R^3$ Wasserstoff, Alkyl, Cycloalkyl, Alkenyl oder Alkinyl
bedeuten.

2. Verbindungen der Formel (I) nach Anspruch 1, dadurch gekennzeichnet, daß
$X^1$ und $X^2$ unabhängig voneinander Halogen oder $C_1$-$C_4$-Halogenalkyl,
$R^1$ $C_1$-$C_6$-Alkyl, $C_3$-$C_6$-Cycloalkyl oder ($C_1$-$C_6$-Alkyloxy)-$C_1$-$C_6$-alkyl,
$R^2$ $C_1$-$C_6$-Alkyl, $C_3$-$C_6$-Cycloalkyl oder $C_1$-$C_6$-Halogenalkyl und
$R^3$ Wasserstoff, $C_1$-$C_6$-Alkyl, $C_3$-$C_6$-Cycloalkyl, $C_2$-$C_6$-Alkenyl oder $C_2$-$C_6$-Alkinyl
bedeuten.

3. Verbindungen nach Anspruch 1 oder 2, dadurch gekennzeichnet, daß
$X^1$ und $X^2$ unabhängig voneinander Fluor, Chlor, Brom oder Trifluormethyl,
$R^1$ $C_1$-$C_4$-Alkyl, ($C_1$-$C_4$-Alkyloxy)-$C_1$-$C_4$-alkyl,
$R^2$ $C_1$-$C_4$-Alkyl, das unsubstituiert oder ein- oder mehrfach durch Halogen substituiert ist, und
$R^3$ Wasserstoff, $C_1$-$C_4$-Alkyl, $C_2$-$C_4$-Alkenyl oder $C_2$-$C_4$-Alkinyl
bedeuten.

4. Verfahren zur Herstellung von Verbindungen der Formel (I) nach Anspruch 1, dadurch gekennzeichnet, daß man eine Verbindung der Formel II

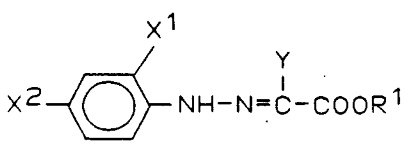

(II)

worin Y für Chlor oder Brom steht und $X^1$, $X^2$ und $R^1$ die oben angegebenen Bedeutungen haben, mit Enolethern der Formel (III)

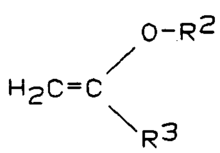

(III)

worin $R^2$ und $R^3$ die oben angegebenen Bedeutungen haben, umsetzt.

5. Pflanzenschützendes Mittel, dadurch gekennzeichnet, daß es eine Verbindung der Formel (I) nach einem oder mehreren der Ansprüche 1 bis 3 und inerte Trägerstoffe oder andere übliche Formulierungshilfsmittel enthält.

**6.** Herbizides Mittel, dadurch gekennzeichnet, daß es mindestens eine Verbindung der Formel (I) nach einem oder mehreren der Ansprüche 1 bis 3 als Safener und ein Herbizid enthält.

**7.** Herbizides Mittel nach Anspruch 6, dadurch gekennzeichnet, daß es zusätzlich inerte Trägerstoffe oder andere übliche Formulierungshilfsmittel enthält.

**8.** Herbizides Mittel nach Anspruch 6 oder 7, dadurch gekennzeichnet, daß es ein Herbizid aus der Gruppe der Carbamate, Thiocarbamate, Halogenacetanilide, substituierte Phenoxy-, Naphthoxy- und Phenoxyphenoxycarbonsäurederivate, Heteroaryloxyphenoxycarbonsäurederivate und Dimedonabkömmlinge enthält.

**9.** Verfahren zum Schutz von Kulturpflanzen gegen phytotoxische Nebenwirkungen von Herbiziden, dadurch gekennzeichnet, daß man die Pflanzen, Pflanzensamen oder Anbauflächen mit einer Verbindung der Formel (I) nach Anspruch 1, 2 oder 3, vor, nach oder gleichzeitig mit dem Pflanzenschutzmittel behandelt.

**10.** Verwendung einer Verbindung nach einem oder mehreren der Ansprüche 1 bis 3 als Safener gegen phytotoxische Nebenwirkungen von Pflanzenschutzmitteln.

**11.** Verwendung von Verbindungen der Formel (I) nach einem oder mehreren der Ansprüche 1 bis 3 als Zwischenprodukt zur Herstellung von pflanzenschützenden Pyrazolen der Formel

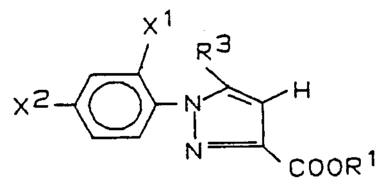

worin
$X^1$, $X^2$, $R^1$ und $R^3$ wie in Formel (I) definiert sind.

**12.** Verfahren zur Herstellung von pflanzenschützenden Pyrazolen der Formel

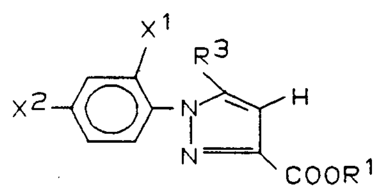

worin
$X^1$, $X^2$, $R^1$ und $R^3$ die in Formel (I) nach Anspruch 1 definierte Bedeutung haben, dadurch gekennzeichnet, daß die Verbindungen der Formel (I) ohne oder in Gegenwart eines organischen Lösungsmittels ohne oder in Gegenwart einer anorganischen oder organischen Säure bei Temperaturen zwischen 0 und 200 °C unter Abspaltung der Verbindung der Formel $HOR^2$, worin $R^2$ die in Formel (I) definierte Bedeutung hat, behandelt werden.

**Patentansprüche für folgenden Vertragsstaat : ES**

1. Verwendung von Verbindungen der Formel (I)

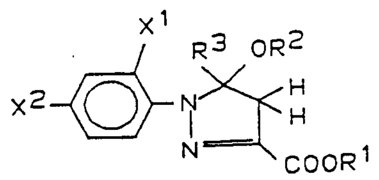

(I)

worin

| | |
|---|---|
| $X^1$ und $X^2$ | unabhängig voneinander Halogen oder Halogenalkyl, |
| $R^1$ | Wasserstoff, Alkyl, Cycloalkyl oder Alkoxyalkyl, |
| $R^2$ | Alkyl, Cycloalkyl oder Halogenalkyl und |
| $R^3$ | Wasserstoff, Alkyl, Cycloalkyl, Alkenyl oder Alkinyl |

bedeuten, als Safener gegen phytotoxische Nebenwirkungen von Herbiziden.

2. Verwendung nach Anspruch 1, dadurch gekennzeichnet, daß

| | |
|---|---|
| $X^1$ und $X^2$ | unabhängig voneinander Halogen oder $C_1$-$C_4$-Halogenalkyl, |
| $R^1$ | $C_1$-$C_6$-Alkyl, $C_3$-$C_6$-Cycloalkyl oder ($C_1$-$C_6$-Alkyloxy)-$C_1$-$C_6$-alkyl, |
| $R^2$ | $C_1$-$C_6$-Alkyl, $C_3$-$C_6$-Cycloalkyl oder $C_1$-$C_6$-Halogenalkyl und |
| $R^3$ | Wasserstoff, $C_1$-$C_6$-Alkyl, $C_3$-$C_6$-Cycloalkyl, $C_2$-$C_6$-Alkenyl oder $C_2$-$C_6$-Alkinyl |

bedeuten.

3. Verwendung nach Anspruch 1 oder 2, dadurch gekennzeichnet, daß

| | |
|---|---|
| $X^1$ und $X^2$ | unabhängig voneinander Fluor, Chlor, Brom oder Trifluormethyl, |
| $R^1$ | $C_1$-$C_4$-Alkyl, ($C_1$-$C_4$-Alkyloxy)-$C_1$-$C_4$-alkyl, |
| $R^2$ | $C_1$-$C_4$-Alkyl, das unsubstituiert oder ein- oder mehrfach durch Halogen substituiert ist, und |
| $R^3$ | Wasserstoff, $C_1$-$C_4$-Alkyl, $C_2$-$C_4$-Alkenyl oder $C_2$-$C_4$-Alkinyl |

bedeuten.

4. Verfahren zur Herstellung von Verbindungen der Formel (I) nach Anspruch 1, dadurch gekennzeichnet, daß man eine Verbindung der Formel (II)

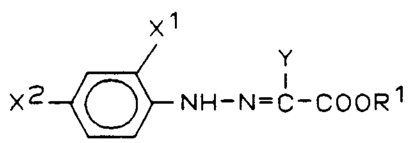

(II)

worin Y für Chlor oder Brom steht und $X^1$, $X^2$ und $R^1$ die oben angegebenen Bedeutungen haben, mit Enolethern der Formel (III)

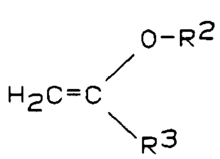

(III)

worin $R^2$ und $R^3$ die oben angegebenen Bedeutungen haben, umsetzt.

5. Pflanzenschützendes Mittel, dadurch gekennzeichnet, daß es eine Verbindung der Formel (I) nach einem oder mehreren der Ansprüche 1 bis 3 und inerte Trägerstoffe oder andere übliche Formulierungshilfsmittel enthält.

6. Verfahren zur Herstellung eines pflanzenschützenden Mittels, dadurch gekennzeichnet, daß man eine Verbindung der Formel (I) nach einem oder mehreren der Ansprüche 1 bis 3 nach üblichen Methoden mit inerten Trägerstoffen oder anderen üblichen Formulierungshilfsmitteln formuliert.

7. Herbizides Mittel, dadurch gekennzeichnet, daß es mindestens eine Verbindung der Formel (I) nach einem oder mehreren der Ansprüche 1 bis 3 als Safener und ein Herbizid enthält.

8. Verfahren zur Herstellung eines selektiven herbiziden Mittels, dadurch gekennzeichnet, daß man eine Verbindung der Formel (I) nach einem oder mehreren der Ansprüche 1 bis 3 zusammen mit einem Herbizid nach üblichen Methoden formuliert.

9. Herbizides Mittel nach Anspruch 7 oder 8, dadurch gekennzeichnet, daß es ein Herbizid aus der Gruppe der Carbamate, Thiocarbamate, Halogenacetanilide, substituierte Phenoxy-, Naphthoxy- und Phenoxyphenoxycarbonsäurederivate, Heteroaryloxyphenoxycarbonsäurederivate und Dimedonabkömmlinge enthält.

10. Verfahren zum Schutz von Kulturpflanzen gegen phytotoxische Nebenwirkungen von Herbiziden, dadurch gekennzeichnet, daß man die Pflanzen, Pflanzensamen oder Anbauflächen mit einer Verbindung der Formel (I) nach Anspruch 1, 2 oder 3, vor, nach oder gleichzeitig mit dem Pflanzenschutzmittel behandelt.

11. Verwendung von Verbindungen der Formel (I) nach einem oder mehreren der Ansprüche 1 bis 3 als Zwischenprodukt zur Herstellung von pflanzenschützenden Pyrazolen der Formel

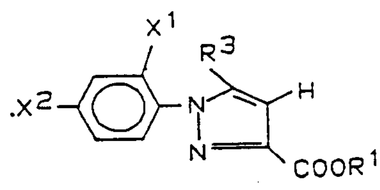

worin
$X^1$, $X^2$, $R^1$ und $R^3$ wie in Formel (I) definiert sind.

12. Verfahren zur Herstellung von pflanzenschützenden Pyrazolen der Formel

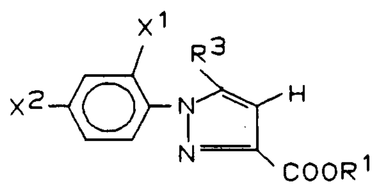

worin
$X^1$, $X^2$, $R^1$ und $R^3$ die in Formel (I) nach Anspruch 1 definierte Bedeutung haben, dadurch gekennzeichnet, daß die Verbindungen der Formel (I) ohne oder in Gegenwart eines organischen Lösungsmittels ohne oder in Gegenwart einer anorganischen oder organischen Säure bei Temperaturen zwischen 0 und 200 °C unter Abspaltung der Verbindung der Formel $HOR^2$, worin $R^2$ die in Formel (I) definierte Bedeutung hat, behandelt werden.

## Claims

**Claims for the following Contracting States : BE, CH, DE, FR, GB, IT, LI, NL**

1. A compound of the formula (I)

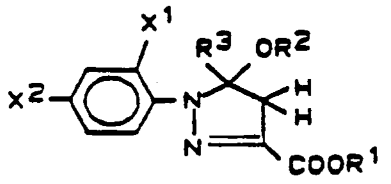

(I)

where
$X^1$ and $X^2$ independently of one another are halogen or haloalkyl,
$R^1$ is hydrogen, alkyl, cycloalkyl or alkyloxalkyl,
$R^2$ is alkyl, cycloalkyl or haloalkyl and
$R^3$ is hydrogen, alkyl cycloalkyl, alkenyl or alkynyl.

2. A compound of the formula (I) as claimed in claim 1, wherein
$X^1$ and $X^2$ independently of one another are halogen or $C_1$-$C_4$-haloalkyl,
$R^1$ is $C_1$-$C_6$-alkyl, $C_3$-$C_6$-cycloalkyl or ($C_1$-$C_6$-alkyloxy)-$C_1$-$C_6$-alkyl,
$R^2$ is $C_1$-$C_6$-alkyl, $C_3$-$C_6$-cycloalkyl or $C_1$-$C_6$-haloalkyl and
$R^3$ is hydrogen, $C_1$-$C_6$-alkyl, $C_3$-$C_6$-cycloalkyl $C_2$-$C_6$-alkenyl or $C_2$-$C_6$-alkynyl.

3. A compound as claimed in claim 1 or 2, wherein
$X^1$ and $X^2$ independently of one another are fluorine, chlorine, bromine or trifluoromethyl,
$R^1$ is $C_1$-$C_4$-alkyl or ($C_1$-$C_4$-alkyloxy)-$C_1$-$C_4$-alkyl,
$R^2$ is $C_1$-$C_4$-alkyl which is unsubstituted or monosubstituted or polysubstituted by halogen, and
$R^3$ is hydrogen, $C_1$-$C_4$-alkyl, $C_2$-$C_4$-alkenyl or $C_2$-$C_4$-alkynyl.

4. A process for the preparation of a compound of the formula (I) as claimed in claim 1, which comprises reacting a compound of the formula (II)

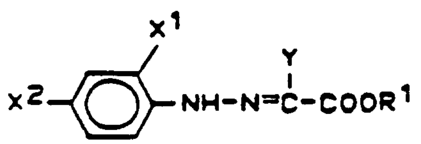

(II)

where Y is chlorine or bromine and $X^1$, $X^2$ and $R^1$ are as defined above, with enol ethers of the formula (III)

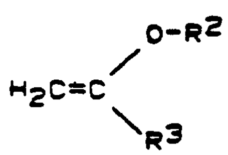

(III)

where $R^2$ and $R^3$ have the abovementioned meanings.

5. A plant-protecting agent which contains a compound of the formula (I), as claimed in one or more of claims 1 to 3, and inert carriers or other customary formulation auxiliaries.

6. A herbicidal agent which contains at least one compound of the formula (I), as claimed in one or more of claims 1 to 3, as safener, and a herbicide.

**7.** A herbicidal agent as claimed in claim 6, which contains, in addition, inert carriers or other customary formulation auxiliaries.

**8.** A herbicidal agent as claimed in claim 6 or 7, which contains a herbicide from the group of the carbamates, thiocarbamates, haloacetanilides, substituted phenoxy-, naphthoxy- and phenoxyphenoxycarboxylic acid derivatives, heteroaryloxyphenoxycarboxylic acid derivatives and dimedon-derived substances.

**9.** A process for protecting crop plants against phytotoxic secondary effects of herbicides, which comprises treating the plants, seeds of the plants or areas under cultivation with a compound of the formula (I) as claimed in claim 1, 2 or 3, prior to, after, or simultaneously with, the plant protection agent.

**10.** The use of a compound as claimed in one or more of claims 1 to 3 as safener against phytotoxic secondary effects of plant protection agents.

**11.** The use of a compound of the formula (I) as claimed in one or more of claims 1 to 3 as an intermediate for the preparation of plant-protecting pyrazoles of the formula

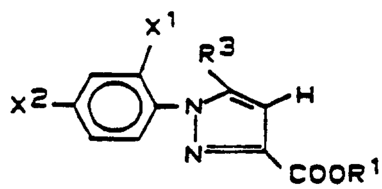

where
$X^1$, $X^2$, $R^1$ and $R^3$ are as defined in formula (I).

**12.** A process for the preparation of plant-protecting pyrazoles of the formula

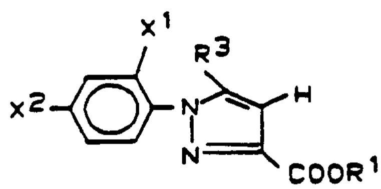

where
$X^1$, $X^2$, $R^1$ and $R^3$ are as defined in formula (I) as claimed in claim 1, which comprises treating the compounds of the formula (I) without or in the presence of an organic solvent, without or in the presence of an inorganic or organic acid, at temperatures between 0 and 200 ° C, during which process the compound of the formula $HOR^2$, where $R^2$ is as defined in formula (I), is eliminated.

**Claims for the following Contracting State : ES**

**1.** The use of a compound of the formula (I)

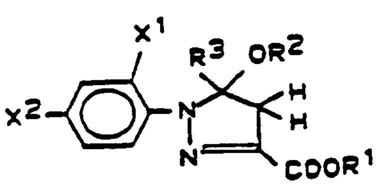

(I)

where
$X^1$ and $X^2$ independently of one another are halogen or haloalkyl,
$R^1$ is hydrogen, alkyl, cycloalkyl or alkyloxyalkyl,
$R^2$ is alkyl, cycloalkyl or haloalkyl and
$R^3$ is hydrogen, alkyl, cycloalkyl, alkenyl or alkynyl, as safener against phytotoxic secondary effects of herbicides.

**2.** The use as claimed in claim 1, wherein
$X^1$ and $X^2$ independently of one another are halogen or $C_1$-$C_4$-haloalkyl,
$R^1$ is $C_1$-$C_6$-alkyl, $C_3$-$C_6$-cycloalkyl or ($C_1$-$C_6$-alkyloxy)-$C_1$-$C_6$-alkyl,
$R^2$ is $C_1$-$C_6$-alkyl, $C_3$-$C_6$-cycloalkyl or $C_1$-$C_6$-haloalkyl and
$R^3$ is hydrogen, $C_1$-$C_6$-alkyl, $C_3$-$C_6$-cycloalkyl, $C_2$-$C_6$-alkenyl or $C_2$-$C_6$-alkynyl.

**3.** The use as claimed in claim 1 or 2, wherein
$X^1$ and $X^2$ independently of one another are fluorine, chlorine, bromine or trifluoromethyl,
$R^1$ is $C_1$-$C_4$-alkyl or ($C_1$-$C_4$-alkyloxy)-$C_1$-$C_4$-alkyl, $R^2$ is $C_1$-$C_4$-alkyl which is unsubstituted or monosubstituted or polysubstituted by halogen, and
$R^3$ is hydrogen, $C_1$-$C_4$-alkyl, $C_2$-$C_4$-alkenyl or $C_2$-$C_4$-alkynyl.

**4.** A process for the preparation of a compound of the formula (I) as claimed in claim 1, which comprises reacting a compound of the formula (II)

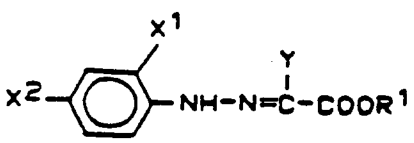

(II)

where Y is chlorine or bromine and $X^1$, $X^2$ and $R^1$ are as defined above, with enol ethers of the formula (III)

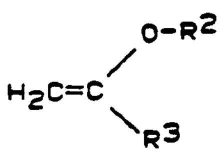

(III)

where $R^2$ and $R^3$ have the abovementioned meanings.

**5.** A plant-protecting agent which contains a compound of the formula (I), as claimed in one or more of claims 1 to 3, and inert carriers or other customary formulation auxiliaries.

**6.** A process for producing a plant-protecting agent, which comprises formulating a compound of the formula (I) as claimed in one or more of claims 1 to 3, by conventional methods, with inert carriers or other customary formulation auxiliaries.

**7.** A herbicidal agent which contains at least one compound of the formula (I), as claimed in one or more of claims 1 to 3, as safener, and a herbicide.

**8.** A process for producing a selective herbicidal agent, which comprises formulating a compound of the formula (I) as claimed in one or more of claims 1 to 3, by conventional methods, together with a herbicide.

**9.** A herbicidal agent as claimed in claim 7 or 8, which contains a herbicide from the group of the carbamates, thiocarbamates, haloacetanilides, substituted phenoxy-, naphthoxy- and phenoxyphenoxycarboxylic acid derivatives, heteroaryloxyphenoxycarboxylic acid derivatives and dimedon-derived

substances.

**10.** A process for protecting crop plants against phytotoxic secondary effects of herbicides, which comprises treating the plants, seeds of the plants or areas under cultivation with a compound of the formula (I) as claimed in claim 1, 2 or 3, prior to, after, or simultaneously with, the plant protection agent.

**11.** The use of a compound of the formula (I) as claimed in one or more of claims 1 to 3 as an intermediate for the preparation of plant-protecting pyrazoles of the formula

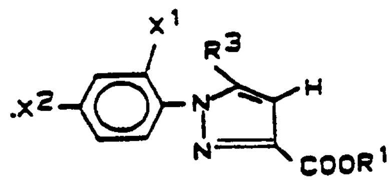

where
$X^1$, $X^2$, $R^1$ and $R^3$ are as defined in formula (I).

**12.** A process for the preparation of plant-protecting pyrazoles of the formula

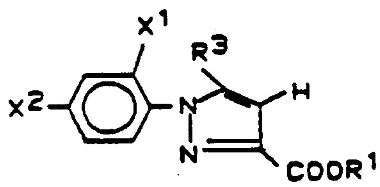

where
$X^1$, $X^2$, $R^1$ and $R^3$ are as defined in formula (I) as claimed in claim 1, which comprises treating the compounds of the formula (I) without or in the presence of an organic solvent, without or in the presence of an inorganic or organic acid, at temperatures between 0 and 200 °C, during which process the compound of the formula $HOR^2$, where $R^2$ is as defined in formula (I), is eliminated.

**Revendications**
**Revendications pour les Etats contractants suivants : BE, CH, DE, FR, GB, IT, LI, NL**

**1.** Composés répondant à la formule (I)

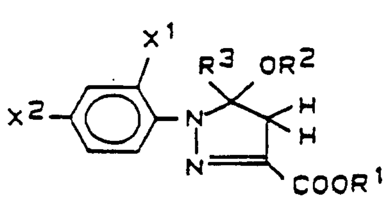

(I)

dans laquelle
$X^1$ et $X^2$ désignent indépendamment l'un de l'autre un atome d'halogène ou un groupe halogénoalkyle,
$R^1$ désigne un atome d'hydrogène, un groupe alkyle, cycloalkyle ou alcoxyalkyle,
$R^2$ désigne un groupe alkyle, cycloalkyle ou halogénoalkyle et
$R^3$ désigne un atome d'hydrogène, un groupe alkyle, cycloalkyle, alcényle ou alcinyle.

**2.** Composés répondant à la formule (I) selon la revendication 1, caractérisés en ce que
$X^1$ et $X^2$ désignent indépendamment l'un de l'autre un atome d'halogène ou un groupe halogénoalkyle

en $C_1$-$C_4$
$R^1$ désigne un groupe alkyle en $C_1$-$C_6$, cycloalkyle en $C_3$-$C_6$ ou (alkyloxy en $C_1$-$C_6$) (alkyle en $C_1$-$C_6$),
$R^2$ désigne un groupe alkyle en $C_1$-$C_6$, cycloalkyle en $C_3$-$C_6$ ou halogénoalkyle en $C_1$-$C_6$ et
$R^3$ désigne un atome d'hydrogène, un groupe alkyle en $C_1$-$C_6$, cycloalkyle en $C_3$-$C_6$, alcényle en $C_2$-$C_6$ ou alcinyle en $C_2$-$C_6$.

3. Composés selon les revendications 1 ou 2, caractérisés en ce que
$X^1$ et $X^2$ désignent indépendamment l'un de l'autre des atomes de fluor, de chlore, de brome ou un groupe trifluorométhyle,
$R^1$ désigne un groupe alkyle en $C_1$-$C_4$, (alkyloxy en $C_1$-$C_4$) (alkyle en $C_1$-$C_4$),
$R^2$ désigne un groupe alkyle en $C_1$-$C_4$, qui peut être non substitué ou substitué une ou plusieurs fois par des atomes d'halogène et
$R^3$ désigne un atome d'hydrogène, un groupe alkyle en $C_1$-$C_4$, alcényle en $C_2$-$C_4$ ou alcinyle en $C_2$-$C_4$.

4. Procédé de préparation de composés répondant à la formule (I) selon la revendication 1, caractérisé en ce que l'on fait réagir un composé répondant à la formule II

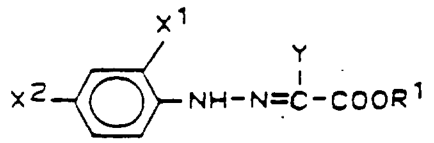

(II)

dans laquelle Y désigne des atomes de chlore et de brome et $X^1$, $X^2$ et $R^1$ ont les significations indiquées ci-dessus, avec des éthers énoliques répondant à la formule III

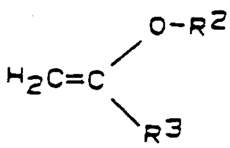

(III)

dans laquelle $R^2$ et $R^3$ ont les significations indiquées ci-dessus.

5. Agent de protection des plantes, caractérisé en ce qu'il contient un composé répondant à la formule I selon une ou plusieurs des revendications 1 à 3 et des supports inertes ou d'autres adjuvants de formulation habituels.

6. Agent herbicide, caractérisé en ce qu'il contient au moins un composé répondant à la formule I selon une ou plusieurs des revendications 1 à 3 comme antidote, et un herbicide.

7. Herbicide selon la revendication 6, caractérisé en ce qu'il contient en outre des supports inertes ou d'autres adjuvants de formulation habituels.

8. Herbicide selon les revendications 6 ou 7, caractérisé en ce qu'il contient un herbicide choisi parmi les carbamates, les thiocarbamates, les halogénoacétanilides, les dérivés d'acides phénoxy-, naphtoxy- et phénoxyphénoxycarboxyliques substitués, des dérivés des acides hétéroaryloxyphénoxycarboxyliques et des dérivés du dimédon.

9. Procédé de protection des plantes cultivées contre les effets secondaires phytotoxiques des herbicides, caractérisé en ce qu'on traite les plantes, les graines des plantes ou les surfaces cultivées par un composé répondant à la formule I selon les revendications 1, 2 ou 3, avant, après ou en même temps que l'agent de protection des plantes.

10. Utilisation d'un composé selon une ou plusieurs des revendications 1 à 3 comme antidote contre les effets secondaires phytotoxiques des agents de protection des plantes.

**11.** Utilisation de composés répondant à la formule I selon une ou plusieurs des revendications 1 à 3 comme produit intermédiaire pour la préparation de pyrazoles protégeant les plantes répondant à la formule

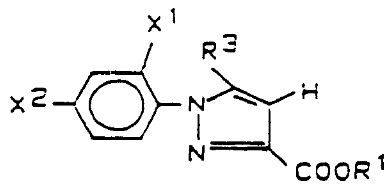

dans laquelle $X^1$, $X^2$, $R^1$ et $R^3$ sont définis comme dans la formule I.

**12.** Procédé de préparation de pyrazoles protégeant les plantes répondant à la formule

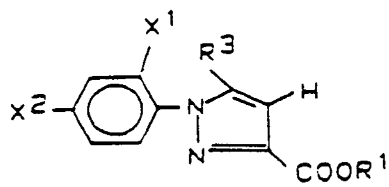

dans laquelle $X^1$, $X^2$, $R^1$ et $R^3$ ont la signification définie dans la formule I, caractérisé en ce qu'on traite les composés répondant à la formule I en présence ou en l'absence d'un solvant organique, en présence ou en l'absence d'un acide minéral ou organique, à des températures comprises entre 0 et 200 °C, avec élimination du composé répondant à la formule $HOR^2$, dans laquelle $R^2$ a la signification définie dans la formule (I).

**Revendications pour l'Etat contractant suivant : ES**

**1.** Utilisation de composés répondant à la formule (I)

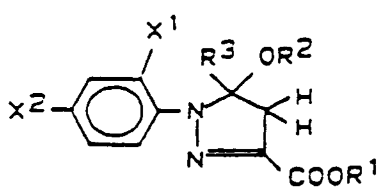

(I)

dans laquelle
$X^1$ et $X^2$ désignent indépendamment l'un de l'autre un atome d'halogène ou un groupe halogénoalkyle,
$R^1$ désigne un atome d'hydrogène, un groupe alkyle, cycloalkyle ou alcoxyalkyle,
$R^2$ désigne un groupe alkyle, cycloalkyle ou halogénoalkyle et
$R^3$ désigne un atome d'hydrogène, un groupe alkyle, cycloalkyle, alcényle ou alcinyle, comme antidote contre les effets secondaires phytotoxiques des herbicides.

**2.** Utilisation selon la revendication 1, caractérisée en ce que
$X^1$ et $X^2$ désignent indépendamment l'un de l'autre un atome d'halogène ou un groupe halogénoalkyle en $C_1$-$C_4$,
$R^1$ désigne un groupe alkyle en $C_1$-$C_6$, cycloalkyle en $C_3$-$C_6$ ou (alkyloxy en $C_1$-$C_6$) (alkyle en $C_1$-$C_6$),
$R^2$ désigne un groupe alkyle en $C_1$-$C_6$, cycloalkyle en $C_3$-$C_6$ ou halogénoalkyle en $C_1$-$C_6$ et
$R^3$ désigne l'atome d'hydrogène, un groupe alkyle en $C_1$-$C_6$, cycloalkyle en $C_3$-$C_6$, alcényle en $C_2$-$C_6$ ou alcinyle en $C_2$-$C_6$.

**3.** Utilisation selon la revendication 1 ou 2, caractérisé en ce que
$X^1$ et $X^2$ désignent indépendamment l'un de l'autre les atomes de fluor, de chlore, de brome ou le groupe trifluorométhyle,
$R^1$ désigne un groupe alkyle en $C_1$-$C_4$, (alkyloxy en $C_1$-$C_4$) (alkyle en $C_1$-$C_4$),
$R^2$ désigne un groupe alkyle en $C_1$-$C_4$, qui peut être non substitué ou substitué une ou plusieurs fois par des atomes d'halogène et
$R^3$ désigne un atome d'hydrogène, un groupe alkyle en $C_1$-$C_4$, alcényle en $C_2$-$C_4$ ou alcinyle en $C_2$-$C_4$.

**4.** Procédé de préparation de composés répondant à la formule (I) selon la revendication 1, caractérisé en ce que l'on fait réagir un composé répondant à la formule II

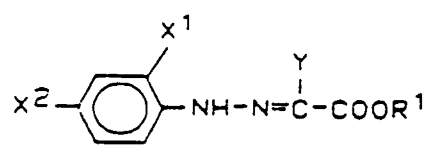

(II)

dans laquelle Y désigne l'atome de chlore ou de brome et $X^1$, $X^2$ et $R^1$ ont les significations indiquées ci-dessus, avec des éthers énoliques répondant à la formule III

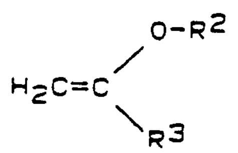

(III)

dans laquelle $R^2$ et $R^3$ ont les significations indiquées ci-dessus.

**5.** Agent de protection des plantes, caractérisé en ce qu'il contient un composé répondant à la formule I selon une ou plusieurs des revendications 1 à 3 et des supports inertes ou d'autres adjuvants de formulation habituels.

**6.** Procédé de préparation d'un agent de protection des plantes, caractérisé en ce que l'on formule un composé répondant à la formule I selon une ou plusieurs des revendications 1 à 3 par des procédés habituels, avec des supports inertes ou d'autres adjuvants de formulation habituels.

**7.** Agent herbicide caractérisé en ce qu'il contient au moins un composé répondant à la formule I selon une ou plusieurs des revendications 1 à 3 comme antidote, et un herbicide.

**8.** Procédé de préparation d'un agent herbicide sélectif, caractérisé en ce que l'on formule un composé répondant à la formule (I) selon une ou plusieurs des revendications 1 à 3 en même temps qu'un herbicide, par les procédés habituels.

**9.** Agent herbicide selon les revendications 7 ou 8, caractérisé en ce qu'il contient un herbicide choisi parmi les carbamates, les thiocarbamates, les halogénoacétanilides, les dérivés substitués des acides phénoxy-, naphtoxy- et phénoxyphénoxycarboxyliques, les dérivés des acides hétéroaryloxyphénoxycarboxyliques et les dérivés du dimédon.

**10.** Procédé de protection des plantes cultivées contre les effets secondaires phytotoxiques des herbicides, caractérisé en ce qu'on traite les plantes, les graines des plantes ou les surfaces cultivées par un composé répondant à la formule (I) selon les revendications 1, 2 ou 3, avant, après ou en même temps que l'agent de protection des plantes.

**11.** Utilisation de composés répondant à la formule (I) selon une ou plusieurs des revendications 1 à 3 comme produits intermédiaires pour la préparation de pyrazoles protégeant les plantes répondant à la formule

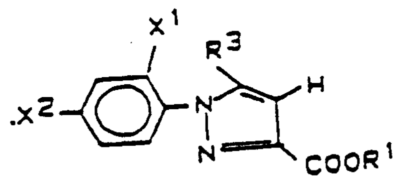

dans laquelle $X^1$, $X^2$, $R^1$ et $R^3$ sont définis comme dans la formule (I).

**12.** Procédé de préparation de pyrazoles protégeant les plantes répondant à la formule

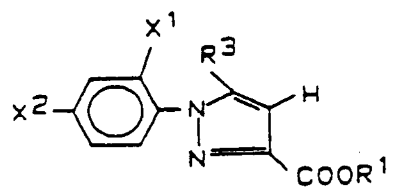

dans laquelle $X^1$, $X^2$, $R^1$ et $R^3$ ont la signification définie dans la formule (I) selon la revendication 1, caractérisé en ce que les composés répondant à la formule (I) sont traités en présence ou en l'absence d'un solvant organique, ou en présence ou en l'absence d'un acide minéral ou organique, à des températures comprises entre 0 et 200 °C, avec élimination du composé répondant à la formule $HOR^2$, dans laquelle $R^2$ a la signification définie dans la formule (I).